# EUROPÄISCHE PATENTSCHRIFT

(11) **EP 0 839 205 B1**
(45) Veröffentlichungstag und Bekanntmachung des Hinweises auf die Patenterteilung: **02.02.2000**
(21) Anmeldenummer: 96923829.4
(22) Anmeldetag: 08.07.1996
(51) Int. Cl.: C12N 15/86, A61K 48/00

(54) **LEBERSPEZIFISCHER ADENOVIRUS-EXPRESSIONSVEKTOR**
LIVER-SPECIFIC ADENOVIRUS EXPRESSION VECTOR
VECTEUR D'EXPRESSION D'ADENOVIRUS HEPATO-SPECIFIQUE

(30) Priorität: 15.07.1995 DE 19525900
(43) Veröffentlichungstag der Anmeldung: 06.05.1998
(73) Patentinhaber: Max-Planck-Gesellschaft zur Förderung der Wissenschaften e.V. Berlin, 80539 München (DE)
(72) Erfinder: SANDIG, Volker, D-13125 Berlin (DE); LÖSER, Peter, D-12437 Berlin (DE); STRAUSS, Michael, D-13187 Berlin (DE)
(74) Vertreter: Baumbach, Friedrich
(86) Internationale Anmeldenummer: DE9601253
(87) Internationale Veröffentlichungsnummer: WO9704117

(56) Entgegenhaltungen:
- WO-A-94/10322
- WO-A-95/16772
- DE-C- 4 339 922
- JOURNAL OF VIROLOGY, Bd. 66, Nr. 12, Dezember 1992, Seiten 7452-7460, XP000610879 SHIN-TAI CHEN ET AL.: "Repression of liver-specific Hepatitis B virus enhancer 2 activity by Adenovirus E1A proteins"

## Beschreibung

Die Erfindung betrifft einen Adenovirusvektor für die leberspezifische Gentherapie; Anwendungsgebiete sind in der Medizin die Behandlung von Gendefekten und Tumorerkrankungen der Leber und die Molekularbiologie.

In den vergangenen Jahren sind zahlreiche Methoden und Vektoren für die Gentherapie entwickelt worden (Übersicht in Mulligan/1993/Science 260, 920). Von Viren abgeleitete Vektoren werden dabei nichtviralen Transferverfahren gegenübergestellt, bei denen das therapeutische Gen in Protein- bzw. Lipidhüllen eingebettet ist. Die Kopplung dieser Partikel an Liganden spezifischer Rezeptoren ermöglicht die bevorzugte Aufnahme durch Zellen, die diese Rezeptoren tragen. Dies ist für den gentherapeutischen Ansatz *in vivo* von Vorteil - trotz systemischer Gabe gelangt das therapeutische Gen nur in Gewebe, in denen dessen Aktivität gewünscht wird.
Dennoch werden gegenwärtig virale Vektoren, vor allem die Virusgruppen Retrovius und Adenovirus, aufgrund ihrer größeren Effektivität *in vivo* bevorzugt. Beide Viren erlauben den Gentransfer in Leberzellen. Die retrovirale Infektion führt zu einer stabilen Integration des genetischen Materials in das zelluläre Genom. Dieser Prozess ist auf Zellproliferation angewiesen - ein für Leberzellen *in vivo* seltenes Ereignis. Während Hepatozyten in Kultur effektiv infiziert werden, erfordert der Einsatz von Retroviren in der Leber deshalb einen Proliferationsstimulus durch Leberteilresektion (Ferry et al./1991/ Proc. Natl. Acad. Sci. USA 88, 8377) oder Verfahren, die zu einem akuten Absterben eines großen Teils der Hepatozyten führen (Lieber et al. Proc. Natl. Acad. Sci. USA in press).

Adenoviren sind allen anderen Vektortypen in ihrer Effektivität weit überlegen. Nahezu 100% der Hepatozyten können selbst bei intravenöser Gabe vom Virus erreicht werden. Adenoviren liegen in der Zelle als Episom vor.

Im Gegensatz zu Retrovirus-Vektoren enthalten adenovirale Vektoren den größten Teil des viralen Genoms. Bei den ursprünglich verwendeten Vektoren wurde lediglich die E1 Region in die zur Virusvermehrung genutzte Helferzelle (HEK293) ausgelagert und damit eine Replikation des Virus im Zielgewebe verhindert. Weil die Adenovirushülle maximal 105% der Genomgröße (40kb) aufnehmen kann, war die El-Deletion auch für die Insertion neuer Gene essentiell. Zur Erweiterung des Aufnahmevermögens auf maximal 8,3kb wurden zusätzlich Teile der E3-Region deletiert (Bett et al./1994/ Proc. Natl. Acad. Sci. USA 91, 8802). Obwohl die wesentlichsten Transaktivatoren adenoviraler Gene, Produkte der El Region, in der Zielzelle fehlen, werden zusätzlich zum therapeutischen Gen auch virale Gene exprimiert. Die Exposition der entsprechenden Proteine auf der Zelloberfläche führt zur Aktivierung von CD8-positiven T-Zellen und zur Elimination der infizierten Zellen. Durch funktionelles Ausschalten weiterer Transaktivatoren z.B. E2A konnte dieser Effekt wesentlich reduziert werden (Yang et al./1994/Nature Genetics 7, 362 ).

Ein wesentlicher Nachteil existierender Adenovirusvektoren besteht in der fehlenden Gewebespezifität. Adenovirusrezeptoren sind auf einer Vielzahl von Zelltypen vorhanden. Die zusätzliche Kopplung von Liganden spezifischer Rezeptoren an das Virus erscheint deshalb wenig aussichtsreich.

Leberspezifität ist außer durch den Aufnahmemodus auch durch Verwendung leberspezifischer Promotoren möglich. Verschiedene in Hepatozyten aktive zelluläre Promotoren (Albumin- und alphal-Antitrypsin-Promotor) wurden auf ihre Eignung für die Gentherapie in Retroviren untersucht (Rettinger et al./1994/ Proc. Natl. Acad. Sci. USA 91, 1460). DE-C-433992 offenbart einen Leberspezifischen Adenovirus-Expressions vektor mit einen Albumin, PEPCK oder OTC Promotor/Enhancer Element. Aufgrund ihrer Größe sind sie jedoch für die Verwendung in Adenovirus-Vektoren ungeeignet. Die im adenoviralen Kontext häufig verwendeten starken viralen Promotoren (CMV- und RSV-Promotor) sind ubiquitär aktiv und werden in der Leber nach kurzer Zeit abgeschaltet.

Ziel der Erfindung ist die Konstruktion eines Vektors auf der Basis von Adenovirus, der die Vorzüge eines Adenovirus-Vektors mit der Eigenschaft einer leberspezifischen Expression von therapeutischen Genen verbindet.

Die Erfindung wird gemäß Anspruch 1 realisiert, die Unteransprüche sind Vorzugsvarianten.

Wesentlicher Bestandteil der Erfindung ist ein kurzer hochaktiver und leberspezifischer Promotor, an welchen das therapeutische Gen gekoppelt wird. Er besteht aus Enhancer-Elementen des Hepatitis B Virus und einem enhancerlosen Minimalpromotor und ist gegebenenfalls von SAR-Elementen (scaffold attached regions) umgeben. Das geschieht, um den Einfluß von adenoviralen Enhancern in der Umgebung des inserierten Gens abzuschirmen. Als ein solches Element wird bevorzugt die SAR-Region des humanen Interferon-β-Gens eingesetzt.

Als Enhancer-Element des Hepatitis B Virus wird bevorzugt der Enhancer II verwendet, er ist durch die Positionen 1628-1807 des viralen Gens bestimmt und auf den Subtyp ayw bezogen.

Als enhancerloser Minimalpromotor wird bevorzugt ein Teil des frühen Promotors des Cytomegalievirus eingesetzt. Er ist durch die Positionen -55 bis +69 hinsichtlich des Transkriptionsstarts gekennzeichnet. Wesentliche Elemente des Minimalpromotors sind die TATA-Box, der "cap-Ort" und die 5'-nichttranslatierte Region. Der Minimalpromotor hat eine niedrige Basisexpression und ist durch den Hepatitis B Virus Enhancer II aktivierbar.

Der bevorzugte Ort für die Insertion der erfindungsgemäßen, aus Enhancer, Promotor und Gen bestehenden Expressionseinheit ist die El-Region des Adenogenoms. Als Adenoviren werden vor allem die Subtypen 2 und 5 dieses Virus eingesetzt.

Als therapeutisches Gen wird die cDNA des humanen LDL-Rezeptors verwendet. Dieses Gen ist bei Patienten mit familiärer Hypercholesterinämie mutiert. Der Rezeptor ist besonders stark auf Hepatocyten vertreten; die Therapie erfordert eine dauerhafte hohe Expression in diesen Zellen.

Die Stelle der cDNA des LDL-Rezeptors können die cDNAs anderer Gene einnehmen, wenn diese bei der zu jeweils behandelnden Krankheit defekt und hauptsächlich in der Leber aktiv sind.

Die Vorteile des erfindungsgemäßen Vektors bestehen in der Verbindung einer hohen Effizienz der Infektion mit hoher Spezifität der Expression für Leberzellen. Darüber hinaus bleibt der Promotor in der Leber über lange Zeit aktiv, wenn er in Adenovirus-Vektoren mit reduzierter Immunogenität verwendet wird. Dies ermöglicht seinen Einsatz zu einer effektiveren Therapie angeborener Gendefekte der Leber.

Die Erfindung soll nachfolgend durch Ausführungsbeispiele näher erläutert werden.

### Ausführungsbeispiel

*In vivo*-Strategien der Lebergentherapie erfordern hepatozytenspezifische Vektoren.
Adenoviren sind für einen *in vivo*-Genetransfer aufgrund hoher Titer und Stabilität im Blut besonders geeignet. Die natürlichen Zielzellen für Adenoviren sind die Zellen epitelialer Gewebe. Ein besonderer Tropismus für Hepatozyten besteht nicht. Die Organspezifität muß also durch spezielle Applikationswege oder Veränderungen am Virus selbst herbeigeführt werden.
Aufgrund der Komplexität der Virushülle und der noch ungeklärten Virus-Rezeptor-Interaktion erscheint eine Modifikation von Virusproteinen für die selektive Infektion von Hepatozyten wenig aussichtsreich. Alternativ kann jedoch die Expression des transferierten therapeutischen Gens durch die Verwendung leberspezifischer Promotoren auf Hepatozyten beschränkt werden.
Zelluläre Promotoren mit bekannter Leberspezifität (Albuminpromotor, Alphal-Antitrypsinpromotor) sind aufgrund ihrer Größe nicht für eine Verwendung in adenoviralen Vektoren geeignet bzw. scheiden aufgrund strenger metabolischer Kontrolle (PEPCK-Promotor) aus.
Das Hepatitis B-Virus besitzt ein Genom von 3,2 kb, dessen Gene von 4 verschiedenen Promotoren (preS1-, S-, core- und X-Promotor) gesteuert werden. Sie werden von 2 Enhancern (Enhancer I und II) in unterschiedlichem Maße zusätzlich aktiviert. Alle diese Kontollelemente umfassen nur wenige hundert Basenpaare.

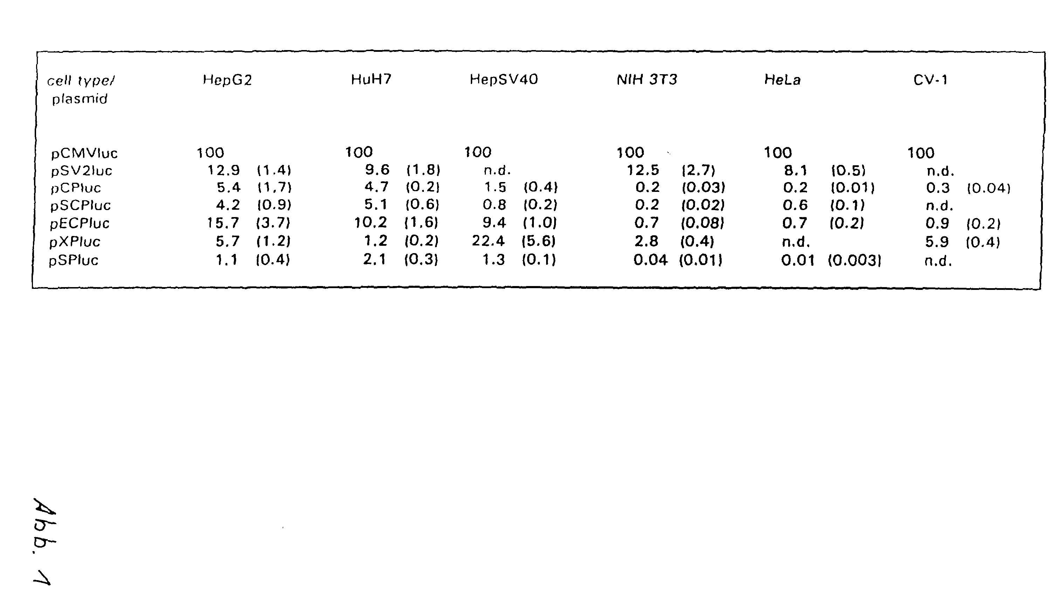

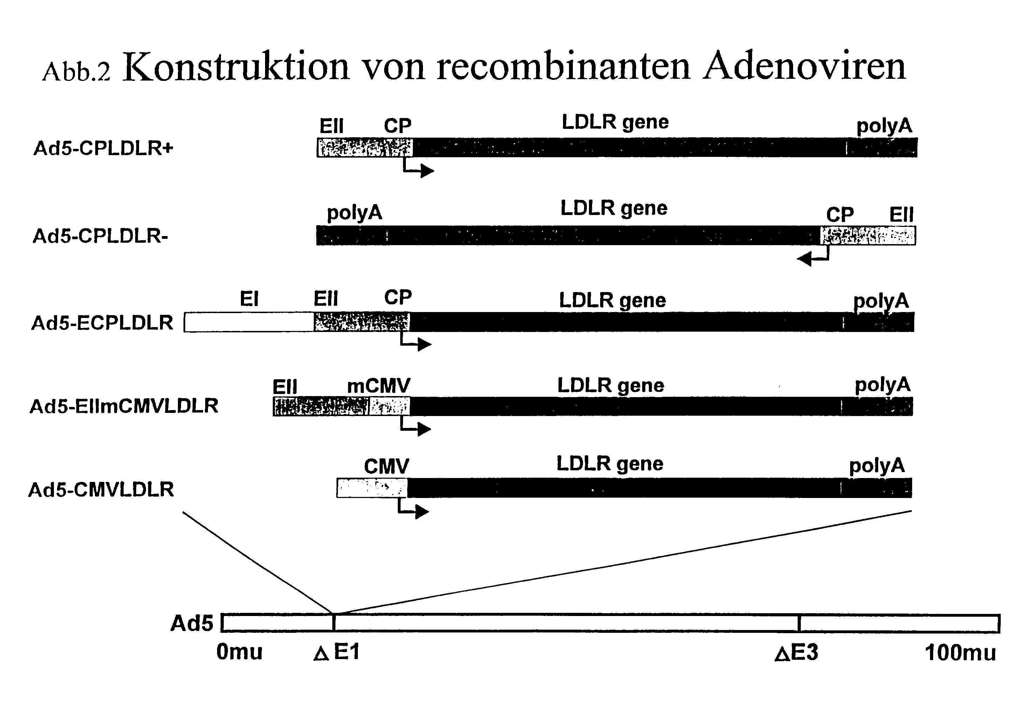

Diese Promotoren/Enhancer wurden durch PCR aus dem klonierten Genom des Hepatitis B-Virus (Subtyp ayw) gewonnen und vor ein promotorloses Luziferasegen kloniert. Im transienten Expressionstest (Bestimmung der Luciferaseaktivität) in den Leberzellinien HuH7 und HepG2 und HepSV40 sowie den Nichtleberzellinien NIH3T3, HeLa und CV1 wurden die Promotoren auf ihre Leberspezifität hin untersucht und zusätzlich die Promotorstärke mit der des CMV-Promotors verglichen. Die Transfektion erfolgte mittels der Ca₃(PO₄)₂-Präzipitationstechnik und wurde durch Kopräzipitation eines RSV-Promotor-gesteuerten β-Galaktosidasegens und Messung von β-Galaktosidaseaktivität standardisiert. (Abb.1)
Mit Hilfe dieser Expressionsstudien wurde der core-Promotor/Enhancer II (pCPluc, nt1628-1807) als Promotor mit deutlicher Preferenz für Hepatozyten bei moderater Promotorstärke ausgewählt.
Es wurde angenommen, daß die Leberspezifität der Expression hauptsächlich durch den Enhancer II vermittelt wird. Um einen leberspezifischen Promotor mit höherer Aktivität zu erzeugen, wurde der Enhancer II mit einem gut aktivierbaren enhancerlosen Minimalpromotor gekoppelt. Als solcher Minimalpromotor wurden TATA-Box und Transkriptionstart-Region (nt-55-+69) des Promotors der frühen Cytomegalievirustranskripte verwendet. Der geschaffene künstliche Promotor (EIImCMV) erreicht in Hepatozyten mehr als 25% der CMV-Promotoraktivität und ist damit als starker Promotor einzustufen. Die Selektivität für Hepatozyten ist weitgehend erhalten. Der Promotor ist auch in primären Hepatozyten von Maus, Schwein und Mensch hochaktiv.
Entscheidend für eine Verwendbarkeit in Gentherapie-Vektoren ist die Langzeitaktivität in Hepatozyten. Für einen diesbezüglichen Test wurden die Promotoren mit dem β-Galaktosidasegen fusioniert und in den episomalen Expressionsvektor pREP8 (Invitrogen) kloniert. Es wurden stabile Hepatozytenlinien (HuH7) mit dem Episom erzeugt und die β-Galaktosidaseexpression über 3 Monate verfolgt. In diesem Zeitraum war kein Absinken der Expression feststellbar.

Die Promotoren CP und EIImCMV sowie der CMV-Promotor wurden im folgenden mit der cDNA des humanen LDL-Rezeptors gekoppelt.

Der Rezeptor wird hauptsächlich in der Leber exprimiert. Die Erbkrankheit "Familiäre Hypercholesterinämie" beruht auf einem Defekt in diesem Gen und ist durch den gezielten Transfer eines aktiven LDL-Rezeptorgens in die Leber therapierbar.

Die Expressionseinheit aus dem jeweiligen Promotor und derLDL-Rezeptor-cDNA wurde in das Adenovirus-Transfer-Plasmid pdElsplA in beiden möglichen Orientierungen inseriert. Durch Kotransfektion mit dem Adenovirusgenom (pJM17) in die Helferzellinie HEK293 wurden rekombinante Adenoviren erzeugt.

Viren aus individuellen Rekombinationsereignissen wurden durch Plaque-Assay isoliert, in HEK293-Zellen vermehrt, durch zweifache Sedimentation im CsCl₂-Gradienten aus dem Zellysat gereinigt und der Titer der Virusstocks bestimmt.

### Testung der Promotoraktivität und Spezifität nach adenoviralem Gentransfer in vitro

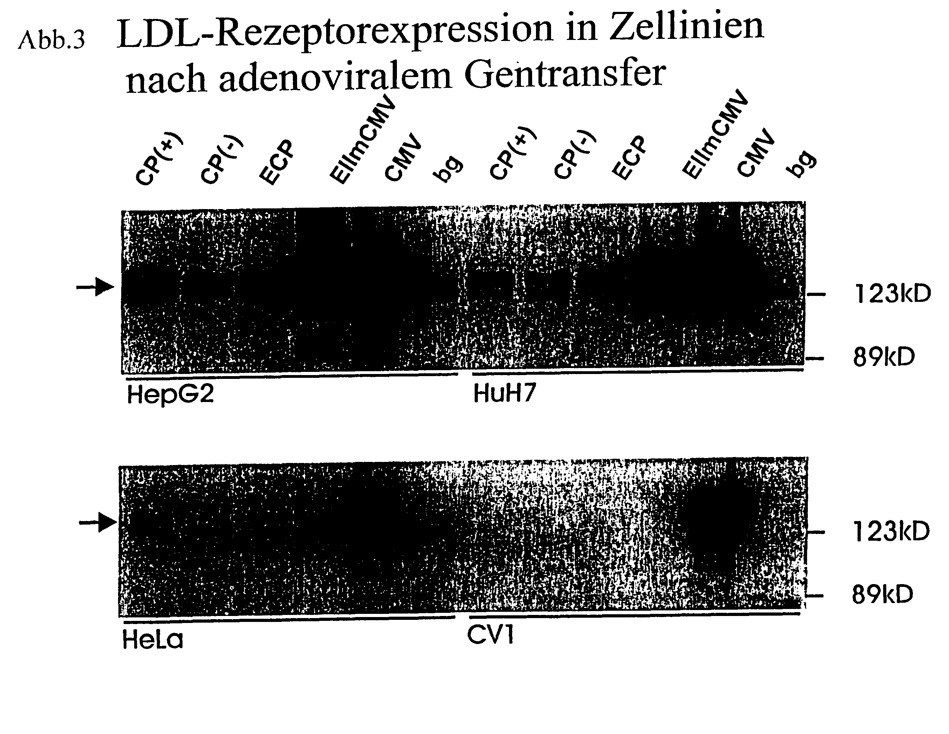

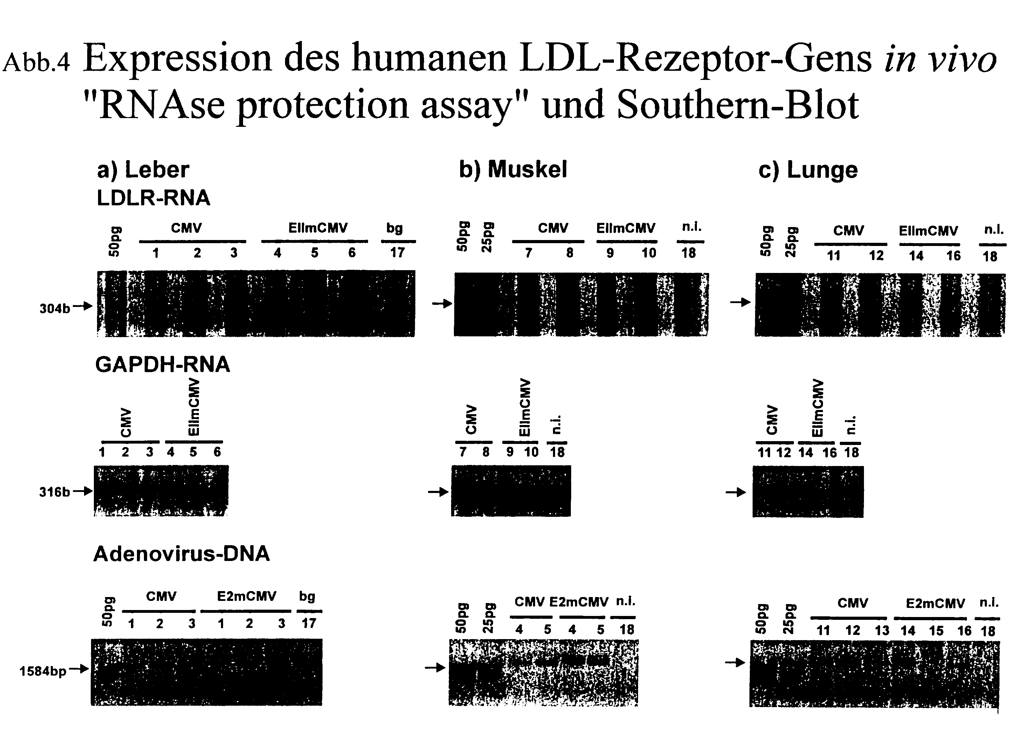

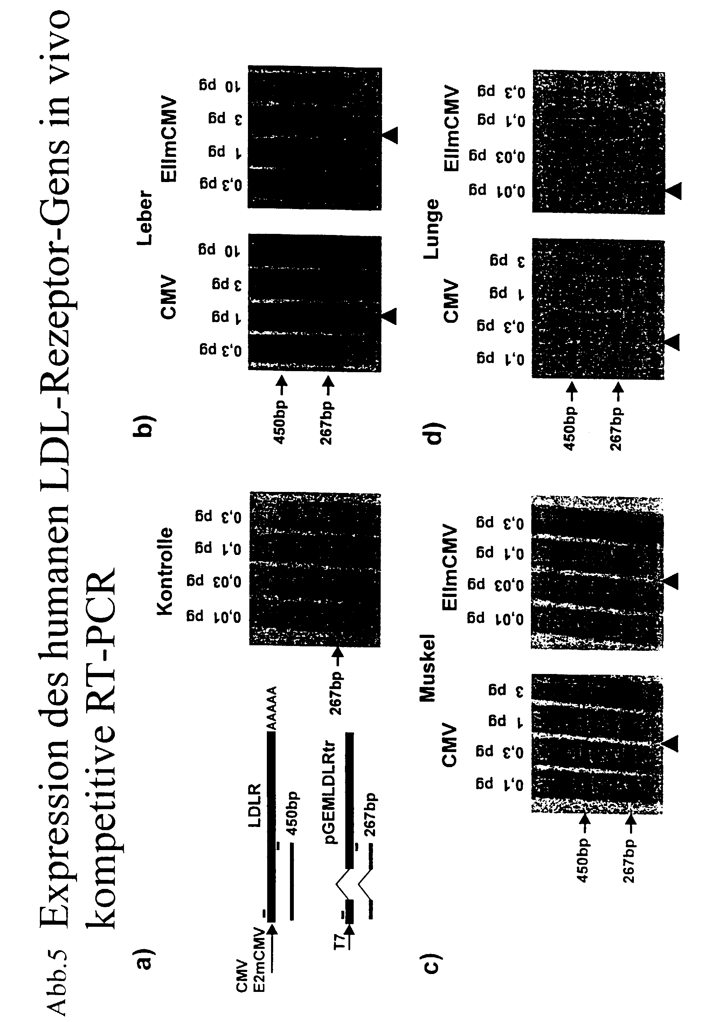

Die Leberzellinien HepG2 und HuH7 sowie die Nichtleberzellinien HeLa und CV1 wurden mit 50 Viren/Zelle infiziert und die Expression des LDL-Rezeptorgens drei Tage nach Infektion auf RNA-Niveau durch RNAse-Schutz sowie auf Protein-Niveau im Western-Blot nachgewiesen.
Während die Aktivität des CMV-Promotor-gesteuerten Gens in allen untersuchten Zellinien eine ähnliche Höhe erreichte,war eine hohe Expression von CP und EIImCMV-Promotoren nur in Hepatozytenzellinien zu finden (Abb.3). Die Orientierung der Expressionseinheit im Virus hatte nur geringfügigen Einfluß auf die Expressionshöhe. Die Aktivität des HBV/CMV-Hybridpromotors erreichte etwa 30% der Aktivität des CMV Promotors.

### Testung der Promotoraktivität und Spezifität in vivo

Um die Aktivität der Promotoren in vivo zu testen, wurden jeweils 2x10⁹ Adenoviren (Ad5-CMVLDLR, Ad5-E2mCMVLDLR oder der KontrollvektorAd5-RSVbg) in die Schwanzvene von Mäusen (Auszuchtstamm NMRI) appliziert. 4 Tage nach Infektion wurden die Tiere getötet und Leber, Lunge, Diaphragma und Niere entnommen. Die Organe wurden in flüssigem Stickstoff pulverisiert und zur Gewinnung von RNA und DNA genutzt. Im Southern-Blot wurde nachgewiesen, daß ein wesentlicher Teil der Viren die Leber infiziert hat. In den anderen Organen wurde nur wenig virale DNA detektiert. Da es aber Aufgabe war, auch geringe Promotoraktivität außerhalb der Leber sicher nachzuweisen, wurde in einer weiteren Gruppe von Mäusen die Lunge durch intranasale Applikation infiziert und in einer 3.Gruppe Virus in den Quadriceps-Muskel injiziert. Tiere, bei denen im Southern-Blot vergleichbare hohe Mengen viraler DNA im jeweils infizierten Organ nachweisbar waren, wurden im "RNAse protection assay" auf Transkripte des humanen LDL-Rezeptors untersucht. Während die Expression vom CMV-Promotor in allen drei Geweben vorhanden war, konnte eine Expression vom E2mCMV Promotor nur in der Leber, nicht aber in Muskel und Lunge nachgewiesen werden. Qualität und Quantität der isolierten RNA wurden durch "RNAse protection assay" für das ubiquitär aktive GAPDH-Gen verifiziert. Da auch der CMV-Promotor nur geringe Aktivität in Lunge und Muskel aufwies, wurde ein hochsensitiver Assay für kompetitive RT-PCR aufgebaut. Dabei werden gleiche Mengen von RNA aus dem jeweiligen Organ mit steigenden Mengen einer verkürzten, in vitro synthetisierten LDL-Rezeptor-RNA (Kompetitor) gemischt, revers transkribiert und in einer PCR ein LDL-Rezeptorfragment amplifiziert. Mit steigender Menge an Kompetitor-RNA nimmt die Stärke des kürzeren PCR-Produkts zu, während die Menge des längeren, zellulärer RNA entsprechenden PCR-Produkts abnimmt. Die Menge an Kompetitor, bei der die Intensität beider Banden m Agarosegel identisch ist, gibt Auskunft über den Gehalt an LDL Rezeptor-RNA im Gewebe.

Die Ergebnisse kompetitiven RT-PCR bestätigten die des "RNAse protection assay". Die Aktivität des E2mCMV-Promotors übersteigt die des CMV-Promotors in der Leber. Sie bleibt aber in Muskel und Lunge um den Faktor 10-30 hinter der des CMV-Promotors zurück.
Es wurden also Adenovirusvektoren konstruiert, die eine hohe Expression des LDL-Rezeptors spezifisch in der Leber selbst bei systemischer Gabe des Virus zulassen. Negative Effekte einer Expression in Zellen außerhalb der Leber werden dadurch ausgeschlossen.

### Legende zu den Abbildungen

### Abb.1

*Expression des Luciferase-Reportergens, gesteuert durch verschiedene Promotoren in Hepatozytenzellinien und Zellinien anderen Ursprungs, gemessen als Aktivität des Luciferase-Enzyms.*

Die Aktivität ist angegeben als % der mit dem CMV Promotor erreichten Aktivität. Die angegebenen Werte sind Mittelwerte aus jeweils 4 unabhängigen Transfektionsexperimenten.

### Abb.2

*Konstruktion von Adenoviren, die das Gen des humanen LDL-Rezeptors unter Kontrolle des CMV- bzw. HBV-abgeleiteter Promotoren exprimieren.*

CMV, CMV immediate early Promotor; CP, HBV core Promotor; EI, HBV Enhancer I; EII, HBV Enhancer II ; mCMV, minimaler CMV Promotor; polyA; Polyadenylationssignal des bovinen Wachstumshormons.

### Abb.3

*LDL-Rezeptorexpression in Zellinien nach adenoviralem Gentransfer*

### Abb.4

*Expression des human LDL Receptor Gens in vivo.*

Proben des jeweiligen Gewebes wurden pulverisiert und gleichermaßen zur Isolation von RNA und genomischer DNA genutzt. Jeweils 10µg Gesamt-RNA wurden im RNA-protection assay eingesetzt. Eine Antisens-RNA des LDL-Rezeptorgens bzw. des GAPDH-Gens, die unter Verwendung ³²P-markierter Nukleotide mittels T7 RNA-Polymerase in vitro synthetisiert wurde, dient dabei als Probe. Sie wird auf einer Länge von 304bp (LDLR) bzw 316bp (GAPDH) vom jeweiligen zellulären Transkript vor RNAse-Verdau geschützt.
Zur Analyse der Infektionseffektivität wurde genomische DNA im Southernblot analysiert. 10 µg genomischer DNA wurden mit Ncol verdaut und im Agarosegel aufgetrennt. Im 5'Bereich des Adenovirus wird dabei ein 1584bp langes Fragment frei, das durch Hybridisierung mit einer ³²P-markierten DNA-Sonde (gleiches Fragment) nachgewiesen wird.

### Abb.5

*Expression des human LDL Receptor Gens in vivo nachgewiesen durch RT-PCR*
a) Schematische Darstellung des Prinzips der kompetitiven RT-PCR. Bei Amplifikation einer revers transkribierten LDLR-RNA aus dem Gewebe entsteht ein 450bp langes Fragment. Aus der in vitro synthetisierten verkürzten LDLR-RNA wird nach reverser Transkription ein 267bp langes Fragment amplifiziert.
b,c,d) Gleiche Mengen von RNA aus dem jeweiligen Gewebe wurden mit steigenden Mengen des verkürzten Transkripts gemischt, revers transkribiert und einer PCR mit ³²P-markierten Nukleotiden über 30 Zyklen unterzogen. Die PCR-Produkte wurden nach Auftrennung im Agarosegel am Phosphoimmager (Fuji) analysiert.
   Die Dreiecke geben das Mischungsverhältnis an, bei dem die PCR-Produkte in equivalenten Mengen vorliegen.

## Patentansprüche

1. Leberspezifischer Adenovirus-Expressionsvektor, gekennzeichnet dadurch, daß ein therapeutisches Gen, das an einen leberspezifischen Promotor gekoppelt ist, welcher aus Enhancer-Elementen des Hepatitis B Virus und einem enhancerlosen Minimalpromotor besteht, in das Adenovirusgenom inseriert wird.

2. Vektor nach Anspruch 1, gekennzeichnet dadurch, daß der Minimalpromotor von SAR-Elementen umgeben ist.

3. Vektor nach Anspruch 1 und 2, gekennzeichnet dadurch, daß als therapeutisches Gen die cDNA eines Gens verwendet wird, das bei der zu behandelnden Krankheit defekt ist.

4. Vektor nach Anspruch 1 bis 3, gekennzeichnet dadurch, daß als therapeutisches Gen die cDNA des humanen LDL-Rezeptors verwendet wird.

5. Vektor nach Anspruch 1-4, gekennzeichnet dadurch, daß als Enhancer-Element der Enhancer II des Hepatitis B Virus verwendet wird.

6. Vektor nach Anspruch 1-5, gekennzeichnet dadurch, daß als Enhancer-Element die Position 1628-1807 des viralen Gens, Subtyp ayw, verwendet wird

7. Vektor nach Anspruch 1-6, gekennzeichnet dadurch, daß als enhancerloser Minimalpromotor ein Teil des frühen Promotors des Cytomegalievirus verwendet wird.

8. Vektor nach Anspruch 1-7, gekennzeichnet dadurch, daß als enhancerloser Minimalpromotor ein Teil des frühen Promotors des Cytomegalievirus, Position -55 bis Position + 69 hinsichtlich des Transkriptionsstarts, die TATA-Box , den "cap-Ort" und die 5'-nichttranslatierte Region enthaltend, verwendet wird.

9. Vektor nach Anspruch 1-8, gekennzeichnet dadurch, daß die Expressionseinheit aus Enhäncer, Promotor und Gen in die E1-Region des Adenogenoms inseriert wird.

10. Vektor nach Anspruch 9, gekennzeichnet dadurch, daß die Insertion in die E1-Region des Genoms von Adenovirus 2 bzw. 5 erfolgt.

## Claims

1. A liver-specific adenovirus expression vector, characterised in that a therapeutic gene, coupled with a liver-specific promoter comprising enhancer elements of the Hepatitis B virus and an enhancer-free minimal promoter, is inserted into the adenovirus genome.

2. A vector as claimed in Claim 1, characterised in that the minimal promoter is surrounded by SAR elements.

3. A vector as claimed in Claim 1 and 2, characterised in that the cDNA of a gene, which is defective in the illness to be treated, is used as therapeutic gene.

4. A vector as claimed in Claims 1 to 3, characterised in that the enhancer II of the human LDL receptor is used as a therapeutic gene.

5. A vector as claimed in Claims 1 to 4, characterised in that the enhancer II of the Hepatitis B virus is used as an enhancer element.

6. A vector as claimed in Claims 1 to 5, characterised in that the position 1628-1807 of the viral gene, subtype ayw, is used as enhancer element.

7. A vector as claimed in Claims I to 6. characterised in that a portion of the previous promoter of the cytomegalovirus is used as enhancer-free minimal promoter.

8. A vector as claimed in Claims 1 to 7, characterised in that a portion of the previous promoter of the cytomegalovirus is used as enhancer-free minimal promoter, and position -55 to position +69 is used with respect to the transcription start, with the TATA-box containing the cap point and the 5'-non-translated region.

9. A vector as claimed in Claims 1 to 8. characterised in that the expression unit of enhancer, promoter and gene is inserted into the region of the adenogenome.

10. A vector as claimed in Claim 9, characterised in that the adenovirus 2 or 5 is inserted into the E1 region of the genome.

## Revendications

1. Vecteur d'expression sur adénovirus à spécificité hépatique, caractérisé en ce qu'un gène thérapeutique couplé à un promoteur à spécificité hépatique, composé d'éléments amplificateurs du virus de l'hépatite B et d'un promoteur minimal sans amplificateur, est inséré dans le génome de l'adénovirus.

2. Vecteur selon la revendication 1, caractérisé en ce que le promoteur minimal est entouré d'éléments SAR.

3. Vecteur selon les revendications 1 et 2, caractérisé en ce que le gêne thérapeutique utilisé est l'ADNc d'un gène qui est défectueux dans la maladie à traiter.

4. Vecteur selon les revendications 1 à 3, caractérisé en ce que le gène thérapeutique utilisé est l'ADNc du récepteur de LDL humain.

5. Vecteur selon les revendications 1 à 4, caractérisé en ce que l'élément amplificateur utilisé est l'amplificateur II du virus de l'hépatite B.

6. Vecteur selon les revendications 1 à 5, caractérisé en ce que l'élément amplificateur utilisé est la position 1628-1807 du gène viral, sous-type ayw.

7. Vecteur selon les revendications 1 à 6, caractérisé en ce que le promoteur minimal sans amplificateur utilisé est une partie du promoteur précoce du cytomégalovirus.

8. Vecteur selon les revendications 1 à 7, caractérisé en ce que le promoteur minimal sans amplificateur utilisé est une partie du promoteur précoce du cytomégalovirus, comprenant les positions -55 à +69 pour le démarrage de la transcription, la séquence TATA, le « site CAP » et la région non traduite 5'.

9. Vecteur selon les revendications 1 à 8, caractérisé en ce que l'unité d'expression formée de l'amplificateur, du promoteur et du gène est introduite dans la région El du génome de l'adénovirus.

10. Vecteur selon la revendication 9, caractérisé en ce que l'insertion est réalisée dans la région El du génome de l'adénovirus 2 ou 5.
